# EUROPEAN PATENT SPECIFICATION

(11) **EP 0 956 293 B1**
(45) Date of publication and mention of the grant of the patent: **17.11.2004**
(21) Application number: 97944781.0
(22) Date of filing: 19.08.1997
(51) Int. Cl.: C07K 5/02, A61K 38/04

(54) **THROMBIN INHIBITORS**
THROMBININHIBITOREN
INHIBITEURS DE LA THROMBINE

(30) Priority: 23.08.1996 EP 96202336
(43) Date of publication of application: 17.11.1999
(73) Proprietor: Akzo Nobel N.V., 6824 BM Arnhem (NL)
(72) Inventor: VAN BOECKEL, Constant, Adriaan, Anton, NL-5345 LX Oss (NL); ADANG, Anton, Egbert, Peter, NL-5615 CR Eindhoven (NL); PETERS, Jacobus, Albertus, Maria, NL-5345 DB Oss (NL); REWINKEL, Johannes, Bernardus, Maria, NL-5348 JT Oss (NL)
(74) Representative: Hogenbirk, Marijke
(86) International application number: PCT/EP1997/004579
(87) International publication number: WO 1998/007308

(56) References cited:
- EP-A- 0 672 658
- WO-A-94/29336
- WO-A-96/11697
- WO-A-96/19491
- WO-A-96/40742
- WO-A-97/17363
- LEWIS S D ET AL: "INHIBITION OF THROMBIN BY PEPTIDES CONTAINING LYSYL-X-KETO CARBONYL DERIVATIVES" THROMBOSIS AND HAEMOSTASIS, vol. 74, no. 4, 1 October 1995, pages 1107-1112, XP000569714 cited in the application
- JONES D M ET AL: "THROMBIN INHIBITORS BASED ON KETONE DERIVATIVES OF ARGININE AND LYSINE" JOURNAL OF ENZYME INHIBITION, vol. 9, 1 January 1995, pages 43-60, XP000570641 cited in the application
- M.J.COSTANZO E.A.: "Potent thrombin inhibitors that probe the S1' subsite: tripeptide transition state analogues based on a heterocycle-activated carbonyl group" JOURNAL OF MEDICINAL CHEMISTRY, vol. 39, no. 16, 2 August 1996, WASHINGTON US, pages 3039-3043, XP002024065

## Description

The invention relates to thrombin inhibitors, pharmaceutical compositions containing the same, as well as the use of said inhibitors for treating and preventing thrombin-related diseases.

Thrombin is a member of the class of the serine proteases. Serine proteases are enzymes which, amongst other things, play an important role in the blood coagulation cascade. Other members of this class of proteases are for example trypsin, factors VIIa, IXa, Xa, XIa, XIIa, and protein C.
Thrombin is the serine protease which regulates the last step in the coagulation cascade. The prime function of thrombin is the cleavage of fibrinogen to generate fibrin monomers, which form an insoluble gel by cross-linking. In addition, thrombin regulates its own production by activating factors V and VIII earlier in the cascade. It also has important actions at cellular level, where it acts on specific receptors to cause platelet aggregation, endothelial cell activation and fibroblast proliferation. Thus thrombin has a central regulatory role in haemostasis and thrombus formation. Since inhibitors of thrombin may have a wide range of therapeutical applications, extensive research has been performed in this area.
In the development of synthetic inhibitors of serine proteases, and more specifically of thrombin, the interest in small synthetic peptides, that are recognized by proteolytic enzymes in a manner similar to that of natural substrates, has increased. As a result, new peptide-like inhibitors have been prepared, such as the transition state inhibitors of thrombin.
The search for more effective and more selective thrombin inhibitors continues unabated in order to obtain thrombin inhibitors which can be administered in lower dosages and which have fewer and less severe side effects.
Furthermore, special attention is paid to oral bioavailability. Potent intravenous thrombin inhibitors are clinically effective in acute care settings requiring the treatment of thrombin-related diseases. However, particularly the prevention of thrombin-related diseases such as myocardial infarct, thrombosis and stroke require long-term therapy, preferably by orally dosing of an anticoagulant.

Many of the peptide-like thrombin inhibitors disclosed in prior publications are based on the sequence -D-Phe-Pro-Arg-, see for example compounds as disclosed by Brady et al. [Bioorganic & Medical Chemistry, 3 (1995), 1063-78]. Thrombin inhibitors may also contain lysine side chains instead of arginine, such as other inhibitors disclosed by Brady et al., and further by Jones et al. [J. Enzyme Inhibition, 9 (1995), 43-60] and Lewis et al. [Thrombosis and Haemostasis, 74(4) (1995), 1107-12]. Also thrombin inhibitors having an aminocyclohexyl moiety instead of lysine or arginine side chain are known [WO 94/25051]. Dipeptidyl thrombin inhibitors wherein the basic side chain has been replaced with varying agmatine (1-amino-4-guanidinobutane)-like groups were described in WO 9429336 (Astra Aktiebolag) and in EP 0672658 (Eli Lilly). Compounds which have in addition to an aminocyclohexyl moiety a keto-functionality at the C-terminus are disclosed in WO 9611697 (Merck & Co,Inc.) as thrombin catalytic site inhibitors (also called transition-state-analogues (TSA-type inhibitors)). Further TSA-type inhibitors derived from the basic -D-Phe-Pro-Arg- sequence, were disclosed in WO 9717363 (Akzo Nobel N.V.) as non-slow-binding thrombin inhibitors.
From these and also other references [e.g. US 5,523,308] further a number of variations at the C-terminus of these peptide-like thrombin inhibitors is known. The developments in this field have already improved the understanding of how to modulate the biological properties of this type of thrombin inhibitors. However, although great effort is being spend on finding selective thrombin inhibitors having good oral bioavailability, there are still few transition state thrombin inhibitors known in the art which fulfill these requirements.

It has now been found that compounds having the formula I, 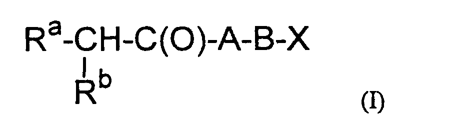 wherein R^{a} is -NR₁R₂ and R^{b} is R₃;
R¹ is -CH₂COOH or -CH₂CH₂COOH;
R² is H;
R³ is -(CH₂)-(3-8C)cycloalkyl;
A is proline, 3,4-dehydroproline or azetidine carboxylic acid;
B is lysine and X is -COOH ; or a pharmaceutically acceptable salt thereof, are potent and selective thrombin inhibitors which show good bioavailability after oral administration.

The compounds of the present invention are useful for treating and preventing thrombin-mediated and thrombin-associated diseases. This includes a number of thrombotic and prothrombotic states in which the coagulation cascade is activated which include, but are not limited to, deep vein thrombosis, pulmonary embolism, thrombophlebitis, arterial occlusion from thrombosis or embolism, arterial reocclusion during or after angioplasty or thrombolysis, restenosis following arterial injury or invasive cardiological procedures, postoperative venous thrombosis or embolism, acute or chronic atherosclerosis, stroke, myocardial infarction, cancer and metastasis, and neurodegenerative diseases. The compounds of the invention may also be used as anticoagulants in extracorporeal blood circuits, as necessary in dialysis and surgery.
The compounds of the invention may also be used as *in vitro* anticoagulants.

Particularly preferred is the compound HOOC-CH₂-D-cyclohexylalanyl-prolyl-lysyl-COOH.

The term (3-8C)cycloalkyl means a cycloalkyl group having 3-8 carbon atoms, being cyclopropyl, cyclobutyl, cyclopentyl, cyclohexyl, cycloheptyl or cyclo-octyl. Cyclopentyl, cyclohexyl and cyclo-octyl are preferred cycloalkyl groups.

The invention further includes a process for preparing a compound of formula I, including coupling of suitably protected amino acids or amino acid analogs, followed by removing the protective groups.
The compounds according to formula I may be prepared in a manner conventional for such compounds. To that end, suitably Nα protected (and side-chain protected if reactive side-chains are present) amino acid derivatives or peptides are activated and coupled to suitably carboxyl protected amino acid or peptide derivatives either in solution or on a solid support. Protection of the α-amino functions generally takes place by urethane functions such as the acid-labile tert-butyloxycarbonyl group (Boc), benzyloxycarbonyl (Cbz) group and substituted analogs or the base-labile 9-fluorenyl-methyloxycarbonyl (Fmoc) group. The Cbz group can also be removed by catalytic hydrogenation. Other suitable amino protective groups include Nps, Bmv, Bpoc, Msc, etc. A good overview of amino protective groups is given is given in The Peptides, Analysis, Synthesis, Biology, Vol. 3 E. Gross and J. Meienhofer, Eds., (Academic Press, New York, 1981). Protection of carboxyl groups can take place by ester formation e.g. base-labile esters like methyl- or ethylesters, acid labile esters like tert-butylesters, or hydrogenolytically-labile esters like benzylesters. Protection of the side chain function of lysine or 4-aminocyclohexylglycine may be accomplished by using the aforementioned groups. Activation of the carboxyl group of the suitably protected amino acids or peptides can take place by the azide, mixed anhydride, active ester, or carbodiimide method, especially with the addition of catalytic and racemization-suppressing compounds like 1-hydroxybenzotriazole, N-hydroxysuccinimide, 3-hydroxy-4-oxo-3,4-dihydro-1,2,3-benzo-triazine, N-hydroxy-5-norbornene-2,3-dicarboximide. See, e.g. The Peptides, Analysis, Synthesis, Biology (*see above*) and Pure and Applied Chem. 59(3), 331-344 (1987).

The compounds of the invention, which can be in the form of a free base, may be isolated from the reaction mixture in the form of a pharmaceutically acceptable salt. The pharmaceutically acceptable salts may also be obtained by treating the free base of formula I with an organic or inorganic acid such as hydrogen chloride, hydrogen bromide, hydrogen iodide, sulfuric acid, phosphoric acid, acetic acid, propionic acid, glycolic acid, maleic acid, malonic acid, methanesulphonic acid, fumaric acid, succinic acid, tartaric acid, citric acid, benzoic acid, and ascorbic acid.

The compounds of this invention possess one or more chiral carbon atoms, and may therefore be obtained as a pure enantiomer, or as a mixture of enantiomers, or as a mixture containing diastereomers. Methods for obtaining the pure enantiomers are well known in the art, e.g. crystallization of salts which are obtained from optically active acids and the racemic mixture, or chromatography using chiral columns. For diastereomers straight phase or reversed phase columns may be used.

The compounds of the invention may be administered enterally or parenterally, and for humans preferably in a daily dosage of 0.001-100 mg per kg body weight, preferably 0.01-10 mg per kg body weight. Mixed with pharmaceutically suitable auxiliaries, e.g. as described in the standard reference, Gennaro et al., Remington's Pharmaceutical Sciences, (18th ed., Mack Publishing Company, 1990, see especially Part 8: Pharmaceutical Preparations and Their Manufacture) the compounds may be compressed into solid dosage units, such as pills, tablets, or be processed into capsules or suppositories. By means of pharmaceutically suitable liquids the compounds can also be applied in the form of a solution, suspension, emulsion, e.g. for use as an injection preparation, or as a spray, e.g. for use as a nasal spray.
For making dosage units, e.g. tablets, the use of conventional additives such as fillers, colorants, polymeric binders and the like is contemplated. In general any pharmaceutically acceptable additive which does not interfere with the function of the active compounds can be used.

Suitable carriers with which the compositions can be administered include lactose, starch, cellulose derivatives and the like, or mixtures thereof, used in suitable amounts.

The invention is further illustrated by the following examples.

### EXAMPLES

*General:* in the examples the following definitions are used:
- Et =: ethyl
- Bzl =: benzyl
- iP =: isopropyl = 1-methylethyl
- Boc =: tert-butyloxycarbonyl
- Cbz =: benzyloxycarbonyl
- Fmoc=: 9-fluorenylmethoxycarbonyl
- Cha =: cyclohexylalanyl
- Coa =: cyclo-octylalanyl
- Lys =: lysyl
- Pro =: prolyl
- DehydroPro =: 3,4-dehydroprolyl
- Azt =: 2-azetidine carboxy

### EXAMPLE 1

### HOOC-CH₂-D-Cha-Pro-LysΨ[COCO]-OH

### (a). H-D-Cha-OMe.HCl

To cold (-20 °C) and dry methanol (195 ml) was added dropwise thionylchloride (28 ml). H-D-Cha-OH.HCl (40 g) was added and the reaction mixture was heated under reflux for 5 h. The mixture was concentrated in vacuo and coevaporated with methanol (3 times). The residue was crystallized from methanol/diethylether yielding H-D-Cha-OMe.HCl as a crystalline powder (40.9 g, 95.8%).
TLC: R_{f}= 0.66, silica gel, n-butanol/acetic acid/water=10/1/3 v/v/v.

### (b). N-(t-Butyloxycarbonylmethyl)-D-Cha-OMe

t-Butyl-bromo acetate (36 g) was added to a stirred solution of H-D-Cha-OMe.HCI (40.9 g) in 400 ml of acetonitrile. The pH of the mixture was adjusted to 8.5 with diisopropylethylamine. The mixture was stirred for 16 hours at room temperature and evaporated in vacuo. The residue was dissolved in dichloromethane and the solution was washed with water, dried on sodium sulfate and evaporated in vacuo. Chromatography over silica gel in heptane/ethyl acetate 9/1 (v/v) gave 64 g of N-(t-butyloxycarbonylmethyl)-D-Cha-OMe.
TLC: R_{f}= 0.25, silica gel, ethyl acetate/heptane=1/1 v/v.

### (c). N-(t-Butyloxycarbonylmethyl)-N-Boc-D-Cha-OMe

sodium sulfate and evaporated in vacuo. Chromatography over silica gel in heptane/ethyl acetate 9/1 (v/v) gave 64 g of N-(t-butyloxycarbonylmethyl)-D-Cha-OMe.
TLC: D_{f}= 0.25, silica gel, ethyl acetate/heptane=1/1 v/v.

### (c). N-(t-Butyloxycarbonylmethyl)-N-Boc-D-Cha-OMe

The pH of a solution of N-(t-butyloxycarbonylmethyl)-D-Cha-OMe (64 g) and di-t-butyl dicarbonate (40.3 g) in 500 ml N,N-dimethylformamide was adjusted to 8.5 with diisopropylethylamine. The mixture was stirred for 16 hours at room temperature. The solvent was removed in vacuo. Dichloromethane and water were added to the residue. The organic layer was separated, washed with cold 1N hydrogen chloride, water, 5% sodium hydrogen carbonate and water. The organic layer was dried on sodium sulfate and the filtrate was evaporated to an amorphous solid ofN-(t-Butyloxycarbonylmethyl)-N-Boc-D-Cha-OMe with a yield of 59.6 g.
TLC: R_{f}=0.50, silica gel, ethyl acetate/heptane=1/1 v/v.

### (d). N-(t-Butyloxycarbonylmethyl)-N-Boc-D-Cha-OH

A solution of N-(t-Butyloxycarbonylmethyl)-N-Boc-D-Cha-OMe (59.6 g) in 900 ml of dioxane/water = 9/1 (v/v) was treated with sufficient 6N sodium hydroxide to keep the pH at 12 for 6 hours at room temperature. After acidification, the mixture was poured into water and was extracted with dichloromethane. The organic layer was washed with water and was dried on sodium sulfate. The filtrate was evaporated and yielded 54 g of N-(t-Butyloxycarbonylmethyl)-N-Boc-D-Cha-OH.
TLC: R_{f}=0.60, silica gel, dichloromethane/methanol = 9/1 v/v.

### (e). N-(t-Butyloxycarbonylmethyl)-N-Boc-D-Cha-Pro-OBzl

To a cold (0 °C) solution of N-(t-Butyloxycarbonylmethyl)-N-Boc-D-Cha-OH (13.5 g) in N,N-dimethylformamide (150 ml) were successively added 1-hydroxy benzotriazole (7.09 g), dicyclohexyl carbodiimide (7.61 g), H-Pro-OBzl.HCl (9.31 g) and triethylamine (6 ml). The mixture was stirred at 0 °C for 1 hour and then kept at room temperature overnight. The mixture was cooled to -20 °C and dicyclohexylurea was removed by filtration. The filtrate was evaporated to dryness. The residue was dissolved in ethyl acetate and washed successively with 5% sodium hydrogen carbonate, water, 3% citric acid and brine, dried over sodium sulfate and concentrated in vacuo. The residue was chromatographed on silica gel in heptane/ethyl acetate = 3/1 (v/v) as eluent. The fractions containing N-(t-Butyloxycarbonylmethyl)-N-Boc-D-Cha-Pro-OBzl were pooled and evaporated. Yield: 15 g.
TLC: R_{f}= 0.70, silica gel, heptane/ethyl acetate = 1/1 v/v.

### (f). N-(t-Butyloxycarbonylmethyl)-N-Boc-D-Cha-Pro-OH

10% palladium on charcoal (750 mg) was added to a solution of N-(t-Butyloxycarbonylmethyl)-N-Boc-D-Cha-Pro-OBzl (15 g) in methanol (150 ml). The mixture was hydrogenated at atmospheric pressure at room temperature for 1 hour. The palladium catalyst was removed by filtration and the solvent was removed by evaporation at reduced pressure yielding 11.2 g N-(t-Butyloxycarbonylmethyl)-N-Boc-D-Cha-Pro-OH.
TLC: R_{f}=0.65, silica gel, ethyl acetate/pyridine/acetic acid/water = 213/20/6/11 v/v/v/v.

### (g). Cbz-Lys(Boc)-OMe

Cbz-Lys(Boc)-OH (28 g) was dissolved in dichloromethane/methanol = 9/1 v/v (500 ml). 2-(1H-benzotriazol-1-yl)-1,1,3,3-tetramethyluronium tetrafluoroborate (23.6 g) was added and the pH of the solution was adjusted to 8 by addition of triethylamine. The reaction mixture was stirred for 2 h at room temperature. The mixture was washed successively with cold 1N hydrogen chloride solution, water, 5% sodium hydrogencarbonate, and water and dried over sodium sulfate. The filtrate was evaporated and the residue was chromatographed on silica gel in ethylacetate/heptane = 1/4 v/v as eluent. The fractions containing Cbz-Lys(Boc)-OMe were pooled and evaporated. Yield: 29.1 g
TLC: R_{f}= 0.85, silica gel, ethyl acetate/heptane=3/1 v/v.

### (h). Cbz-Lys(Boc)Ψ[cyanoacetate]

To a cold (-78 °C) solution of Cbz-Lys(Boc)-OMe (29.1 g) in dry dichloromethane (800 ml) was added dropwise diisobutyl aluminiumhydride (222 ml of 1M solution in hexane) at a rate to keep the reaction temperature below -70 °C. The resulting solution was stirred at -78 °C for 1 h. A 5% citric-acid solution (600 ml) was added to the reaction mixture. The two layer mixture was stirred at room temperature for 10 minutes, the layers were separated and the aqueous layer was extracted twice with dichloromethane. The combined dichloromethane layers were washed with water and dried over sodium sulfate and filtered. The solution was placed under nitrogen and cooled on a icewater-bath. A solution of sodium cyanide (36.3 g) and benzyltriethyl ammonium chloride (4.2 g) in water (600 ml) was added. Under vigorous stirring acetic anhydride was added portionwise (2 x 9 ml) over a period of 30 min. The organic layer was separated and the aqueous layer was extracted twice with dichloromethane. The combined dichloromethane layers were washed with water, dried over sodium sulfate, filtered and evaporated in vacuo. The residue was purified by chromatography on silica (eluent : heptane/ethyl acetate= 1/1 v/v) to yield Cbz-Lys (Boc)Ψ[cyanoacetate] (26.3 g.) .
TLC: Rf= 0.60, silica gel, dichloromethane/ethyl acetate = 7/3 v/v.

### (i). Cbz-Lys(Boc)Ψ[CHOHCO]-OMe

A solution of Cbz-Lys(Boc)Ψ[cyanoacetate] (26.3 g.) in diethylether/methanol = 3/1 v/v (600 ml) was cooled to -20 °C under nitrogen, and 66 g of gaseous hydrochloric acid was introduced keeping the temperature below -5 °C. The reaction mixture was kept at 4 °C overnight. Water (100 ml) was added dropwise to the reaction mixture keeping the temperature below 5 °C. After stirring for 16 h at room temperature the organic layer was separated and washed with water. The aqueous layer was saturated with sodium chloride and extracted with sec-butanol/dichloromethane = 3/2 v/v. The organic phase was washed with brine, dried over sodium sulfate, filtered and evaporated in vacuo to give 25.4 g of the crude amine. The residue was taken up in N,N-dimethyl formamide (400 ml), bis-(tert-butyl)anhydride (16 g) was added and triethylamine until pH 8. The reaction mixture was stirred at room temperature overnight. The solvent was removed by evaporation at reduced pressure. The residue was dissolved in ethyl acetate, washed with water and brine successively, dried over sodium sulfate, filtered and evaporated in vacuo. The residue was purified by chromatography on silica (eluent: ethylacetate/heptane = 4/6 v/v) to yield Cbz-Lys(Boc)Ψ[CHOHCO]-OMe (15.8 g).
TLC: Rf= 0.75, silica gel, ethyl acetate/pyridine/acetic acid/water=63/20/6/11 v/v/v/v.

### (j). H-Lys(Boc)Ψ[CHOHCO]-OMe

10% palladium on charcoal (92 mg) and 2.18 ml of a 1N hydrochloride solution were added to a solution of Cbz-Lys(Boc)Ψ[CHOHCO]-OMe (0.92 g) in N,N-dimethyl formamide (20 ml). The mixture was hydrogenated at atmospheric pressure at room temperature for 3 h. The palladium catalyst was removed by filtration and the solvent was removed by evaporation at reduced pressure yielding H-Lys(Boc)Ψ[CHOHCO]-OMe.HCl quantitatively.
TLC: R_{f}=0.47, silica gel, ethyl acetate/pyridine/acetic acid/water=88/31/18/7 v/v/v/v.

### (k). N-(t-Butyloxycarbonylmethyl)-N-Boc-D-Cha-Pro-Lys(Boc)Ψ[CHOHCO]-OMe

To a cold (0 °C) solution of N-(t-Butyloxycarbonylmethyl)-N-Boc-D-Cha-Pro-OH (1.0 g) in N,N-dimethyl formamide (25 ml) were successively added 1-hydroxy benzotriazole (0.42 g), dicyclohexyl carbodiimide (0.47 g) and, after 30 min, H-Lys(Boc)Ψ [CHOHCO]-OMe.HCl (0.9 g) and triethylamine (0.3 ml). The mixture was stirred at 0 °C for 1 hour and then kept at room temperature overnight. The mixture was cooled to -20 °C and dicyclohexylurea was removed by filtration. The filtrate was evaporated to dryness. The residue was dissolved in ethyl acetate and washed successively with 1N hydrogen chloride, water, 5% sodium hydrogen carbonate, water, dried over sodium sulfate and concentrated in vacuo. The residue was chromatographed on silica gel in heptane/ethyl acetate=3/7 (v/v) as eluent. The fractions containing N-(t-Butyloxycarbonylmethyl)-N-Boc-D-Cha-Pro-Lys(Boc)Ψ[CHOHCO]-OMe were pooled and the solvents were evaporated. Yield: 1.16 g.
TLC: R_{f}= 0.28, silica gel, heptane/ethyl acetate= 3/7 v/v.

### (1). N-(t-Butyloxycarbonylmethyl)-N-Boc-D-Cha-Pro-Lys(Boc)Ψ[CHOHCO]-OH

N-(t-Butyloxycarbonylmethyl)-N-Boc-D-Cha-Pro-Lys(Boc)Ψ[CHOHCO]-OMe (1.16 g) was dissolved in dioxane/water=7/3 v/v (20 ml) and treated with 0.5M sodium hydroxide solution (3 ml) portionwise over 4 h. at room temperature, keeping the pH at 10-10.5. The reaction mixture was diluted with water (20 ml), 2M hydrogen chloride solution was added until pH 2.0 and the water layer was extracted with dichloromethane. The combined organic phases were washed with water, brine and dried over sodium sulfate, filtered and concentrated in vacuo to yield N-(t-Butyloxycarbonylmethyl)-N-Boc-D-Cha-Pro-Lys(Boc)Ψ[CHOHCO]-OH (1.21 g).
TLC: R_{f}=0.05, silica gel, ethyl acetate/heptane = 8/2 v/v.

### (m). N-(t-Butyloxycarbonylmethyl)-N-Boc-D-Cha-Pro-Lys(Boc)Ψ[COCO]-OH

To a solution of N-(t-Butyloxycarbonylmethyl)-N-Boc-D-Cha-Pro-Lys(Boc)Ψ[CHOHCO]-OH (1.21 g) in dry dichloromethane (50 ml) was added 686 mg of periodinane (Dess-Martin reagent). After 1 h stirring at room temperature, 2% sodium thiosulfate solution was added (50 ml) and the mixture was stirred for 30 min at room temperature. The organic layer was separated, washed with water, dried over soudium sulfate, filtered and evaporated in vacuo to give crude N-(t-Butyloxycarbonylmethyl)-N-Boc-D-Cha-Pro-Lys(Boc)Ψ[COCO]-OH (1.38 g).
TLC: R_{f}=0.16, silica gel, ethyl acetate/pyridine/acetic acid/water=163/20/6/1 v/v/v/v.

### (n). HOOC-CH₂-D-Cha-Pro-LysΨ[COCO]-OH

N-(t-Butyloxycarbonylmethyl)-N-Boc-D-Cha-Pro-Lys(Boc)Ψ[COCO]-OH (1.38 g, crude) was treated with 60% trifluoroacetic acid/dichloromethane (20 ml) for 4 h at room temperature. The reaction mixture was concentrated in vacuo and the residue dissolved in water and directly charged onto a preparative HPLC DeltaPak RP-C₁₈ using a gradient elution system of 20% A/80% B to 20% A/50% B/30% C over 45 min at a flow rate of 80 ml/min (A: 0.5M phosphate buffer pH 2.1, B: water, C: acetonitril/water=6/4). Yield: 555 mg of HOOC-CH₂-D-Cha-Pro-LysΨ[COCO]-OH.
Rt(LC): 23.11 min, A 20 %, B 80 %, C 0 % to A 20 %, B 20 %, C 60 % in 40 min.

### EXAMPLE 2.

**HOOC-CH**_{**2**}**-D-Coa-Pro-LysΨ[COCO]-OH**

### (a). N-(t-Butyloxycarbonylmethyl)-N-Boc-D-Coa-Pro-OH

Cyclooctylmethylbromide (27.49 g) and diethyl acetylaminomalonate (29.10 g) were condensed to diethyl 2-acetylamino-2-(cyclooctylmethyl)-malonate (19.60 g). Subsequent acid hydrolyis afforded H-D,L-Coa-OH.HCl (9.83 g). Esterification, alkylation, Boc protection, saponification and subsequent coupling with H-Pro-OBzl, according to examples 1 (a - e), obtained N-(t-Butyloxycarbonylmethyl)-N-Boc-D,L-Coa-Pro-OBzl (19.61 g). The benzyl ester was removed by hydrogenolysis as in example 1 (f), whereafter the L/L-diastereomer was seperated from the D/L-diastereomer by crystalisation from diethyl ether to give the desired compound (9.34 g).
TLC: R_{f}= 0.1, silica gel, heptane/ethyl acetate = 3/1 v/v.

### (b). HOOC-CH₂-D-Coa-Pro-LysΨ[COCO]-OH

N-(t-Butyloxycarbonylmethyl)-N-Boc-D-Coa-Pro-OH (354 mg) was coupled with H-Lys(Boc)Ψ[CHOHCO]-OMe.HCl (243 mg), under the conditions as described in example 1. Silica gel purification in heptane/ethylacetate 2/8 (v/v) afforded 471 mg of the fully protected tripeptide. Saponification, oxidation, deprotection and HPLC purification, under the conditions as described in examples 1, obtained HOOC-CH₂-D-Coa-Pro-LysΨ[COCO]-OH (175 mg).
Rt(LC): 31.39 min, A 20 %, B 80 %, C 0 % to A 20 %, B 20 %, C 60 % in 40 min.

### EXAMPLE 3.

**HOOC-CH**_{**2**}**CH**_{**2**}**-D-Cha-Pro-LysψlCOCOlOH**

### (a). N-(2-(t-Butyloxycarbonyl)ethyl)-N-Boc-D-Cha-OMe

To H-D-Cha-OMe.HCl (7.11 g) in acetonitrile (25 mL) under a nitrogen atmosphere were added N,N-diisopropylethylamine (2.6mL) and tert-butyl acrylate (25 mL) and this stirred suspension was heated at 40 °C keeping the pH of the reaction mixture at eight by addition of N,N-diisopropylethylamine. After four days additional tert-butyl acrylate (5 mL) was added and the reaction mixture was heated at 40 °C for an additional three days keeping the pH at eight.

The reaction mixture was concentrated under reduced pressure. The residue was dissolved in ethyl acetate, washed with water and brine, dried over sodium sulfate and concentrated. The residue was dissolved in N,N-dimethylformamide (10mL), di-tert-butyl dicarbonate (7.86 g) was added and the pH of the solution was adjusted to eight and maintained at this pH using N,N-diisopropylethylamine. After stirring for 30 hours additional di-tert-butyl dicarbonate (1.0 g) was added and the reaction mixture was stirred at room temperature for an additional day. The reaction mixture was concentrated under reduced pressure. The residue was dissolved in ethyl acetate, washed with water and brine, dried over sodium sulfate and concentrated. The residue was purified using column chromatography (silica gel, ethyl acetate/heptane = 1/5 v/v) to yield N-(2-(t-butyloxycarbonyl)ethyl)-N-Boc-D-Cha-OMe (8.78 g).
TLC: R_{f}= 0.7, silica gel, ethyl acetate/heptane=1/2 v/v.

### (b). N-(2-(t-Butyloxycarbonyl)ethyl)-N-Boc-D-Cha-OH

To stirred solution of (N-(2-(t-butyloxycarbonyl)ethyl)-N-Boc-D-Cha-OMe (10.0 g) in dioxane (75 mL) and water (10 mL) at room temperature was added 2N sodium hydroxide at such rate that the pH of the reaction mixture was kept at 12.5. After 6.5 hours the reaction mixture was neutralised and filtered. The filtrate was concentrated to half its volume, cooled on an ice bath, made acid (pH 3) using 4N hydrochloric acid and extracted with ethyl acetate. The ethyl acetate extract was washed with cold hydrochloric acid (pH 3). The aqueous layers were washed with ethyl acetate and the combined ethyl acetate extracts were dried over sodium sulphate and concentrated. The residue was purified using column chromatography (silica gel, ethyl acetate/heptane = 1/1 v/v) to yield (tBuOOC-CH₂CH₂)(Boc)-D-Cha-OH (4.31 g).
TLC: R_{f} = 0.1, silica gel, ethyl acetate/heptanes=1/5 v/v.

### (c). N-(2-(t-Butyloxycarbonyl)ethyl)-N-Boc-D-Cha-Pro-Lys(Boc)Ψ[COCO]-OMe

N-(2-(t-Butyloxycarbonyl)ethyl)-N-Boc-D-Cha-OH (3.14 g) was coupled with H-Pro-OBzl.HCl and hydrogenated using procedures described in example I to give N-(2-(t-butyloxycarbonyl)ethyl)-N-Boc-D-Cha-Pro-OH (3.96 g). This dipeptide (0.46 g) was coupled with H-Lys(Boc)Ψ[CHOHCO]-OMe according to the procedures described in example 1 to give N-(2-(t-butyloxycarbonyl)ethyl)-N-Boc-D-Cha-Pro-Lys(Boc)Ψ[CHOHCO]-OMe. N-(2-(t-Butyloxycarbonyl)ethyl)-N-Boc-D-Cha-Pro-Lys(Boc)Ψ[CHOHCO]-OMe was used in the Dess Martin oxidation as described in example 1 to yield the title compound (0.55 g)
TLC: R_{f}= 0.6, silica gel, ethyl acetate.

### (d) HOOC-CH₂CH₂-D-Cha-Pro-LysΨ[COCO]OH

To a stirred solution of N-(2-(t-butyloxycarbonyl)ethyl)-N-Boc-D-Cha-Pro-Lys(Boc)Ψ[COCO]-OMe (0.55 g) in dichloromethane (6 mL) at room temperature was added trifluoroacetic acid (2 mL). After six hours the solution was concentrated under reduced pressure and once coevaporated with water. Water (17 mL) and methanol (1 mL) were added to the residue, the resulting suspension was filtered and 36 - 38% hydrochloric acid (1 mL) was added to the filtrate. After one week at room temperature the solution was concentrated under reduced pressure, dissolved in water and charged onto a preparative HPLC DeltaPak RP-C₁₈ using a gradient elution system of 20% A/80% B to 20% A/50% B/30% C over 40 min at a flow rate of 80 ml. Yield: 277 mg of HOOC-CH₂-CH₂-D-Cha-Pro-LysΨ[COCO]-OH.
Rt(LC): 22.2 min, A 20 %, B 80 %, C 0 % to A 20 %, B 20 %, C 60 % in 40 min.

### EXAMPLE 4.

### HOOC-CH₂-D-Cha-Azt-LysΨ[COCO]-OH

### (a). N-(t-Butyloxycarbonyl)-Azt-OH

L-azetidine-2-carboxylic acid (2.44 g) was dissolved in 75 ml t-butanol/water=2/1 v/v. After addition of 4 ml 6M NaOH-solution di-t-butyl dicarbonate (5.79 g) was added and the reaction was stirred for 2 h at room temperature. The mixture was diluted with 250 ml of water and extracted with heptane (3 times). After acidification, the mixture was extracted with dichloromethane. The organic layer was washed with water and was dried on sodium sulfate. The filtrate was evaporated and yielded 5 g of N-(t-Butyloxycarbonyl)-Azt-OH.
TLC: R_{f}=0.50, silica gel, ethyl acetate/pyridine/acetic acid/water=163/20/6/11 v/v/v/v.

### (b). N-(t-Butyloxycarbonyl)-Azt-OBzl

N-(t-Butyloxycarbonyl)-Azt-OH (4.6 g) was dissolved in dichloromethane (50 ml). 2-(1H-benzotriazol-1-yl)-1,1,3,3-tetramethyluronium tetrafluoroborate (23.6 g) and benzylalcohol (2.47 g) were added and the pH of the solution was adjusted to 8 by addition of triethylamine. The reaction mixture was stirred for 1 h at room temperature. The mixture was washed successively with cold 1N hydrogen chloride solution, water, 5% sodium hydrogencarbonate, and water and dried over sodium sulfate. The filtrate was evaporated and the residue was chromatographed on silica gel in ethylacetate/heptane = 1/3 v/v as eluent. The fractions containing N-(t-Butyloxycarbonyl)-Azt-OBzl were pooled and evaporated. Yield: 6 g.
TLC: R_{f}= 0.95, silica gel, ethyl acetate/pyridine/acetic acid/water=163/20/6/11 v/v/v/v.

### (c). HCl.H-Azt-OBzl

N-(t-Butylxoycarbonyl)-Azt-OBzl (6 g) was dissolved in 60 ml 3M HCl_{g}/dioxane and stirred for 1 h at room temperature. The reaction mixture was evaporated to dryness yielding 5.26 g of a white solid.
TLC: R_{f}= 0.50, silica gel, ethyl acetate/pyridine/acetic acid/water=63/20/6/11 v/v/v/v.

### (d). HOOC-CH₂-D-Cha-Azt-LysΨ[COCO]-OH

The tripeptide was prepared according to a similar route as described in example 1.
Yield: 120 mg.
Rₜ(LC): 21.09 min.; 20% A/80% B to 20% A/20% B/60% C in 40 min.

### EXAMPLE 5

### HOOC-CH₂-D-Cha-DehydroPro-LysΨ[COCO]-OH

### (a). N-(t-Butyloxycarbonylmethyl)-N-Boc-D-Cha-DehydroPro-OH

The active-ester coupling between N-(t-Butyloxycarbonylmethyl)-N-Boc-D-Cha-OH and H-3,4-DehydroPro-OMe.HCl and the subsequent saponification to yield 2.58 g N-(t-Butyloxycarbonylmethyl)-N-Boc-D-Cha-DehydroPro-OH were done according to the procedures described in example 5.
TLC: R_{f}=0.74, silica gel, ethyl acetate/pyridine/acetic acid/water = 163/20/6/11 v/v/v/v.

### (b). HOOC-CH₂-D-Cha-DehydroPro-LysΨ[COCO]-OH

The active-ester coupling between N-(t-Butyloxycarbonylmethyl)-N-Boc-D-Cha-DehydroPro-OH and H-Lys(Boc)Ψ[CHOHCO]-OMe.HCl, the saponification, the Dess-Martin oxidation, the deprotection and the purification were done according to the procedures described in example 1, yielding 132 mg HOOC-CH₂-D-Cha-DehydroPro-LysΨ[COCO]-OH.
Rt(LC): 23.33 min, 20% A/80% B to 20% A/20% B/60% C in 40 min.

### EXAMPLE 6.

### Anti-thrombin assay

Thrombin (Factor IIa) is a factor in the coagulation cascade.
The anti-thrombin activity of compounds of the present invention was assessed by measuring spectrophotometrically the rate of hydrolysis of the chromogenic substrate s-2238 exterted by thrombin. This assay for anti-thrombin activity in a buffer system was used to assess the IC₅₀-value of a test compound.

Test medium: Tromethamine-NaCl-polyethylene glycol 6000 (TNP) buffer. Reference compound: I2581 (Kabi) Vehicle: TNP buffer. Solubilisation can be assisted with dimethylsulphoxide, methanol, ethanol, acetonitrile or tert.-butyl alcohol which are without adverse effects in concentrations up to 2.5 % in the final reaction mixture.

### Technique

Reagents*: 1. Tromethamine-NaCl (TN) buffer. Composition of the buffer: Tromethamine (Tris) 6.057 g (50 mmol), NaCl 5.844 g (100 mmol), water to 1 1. The pH of the solution is adjusted to 7.4 at 37 °C with HCl (10 mmol·l⁻¹). 2. TNP buffer: Polyethylene glycol 6000 is dissolved in TN buffer to give a concentration of 3 g·l⁻¹ 3. S-2238 solution: One vial S-2238 (25 mg; Kabi Diagnostica, Sweden) is dissolved in 20 ml TN buffer to give a concentration of 1.25 mg·ml⁻¹ (2 mmol·l⁻¹). 4. Thrombin solution: Human thrombin (16 000 nKat·vial⁻¹; Centraal Laboratorium voor Bloedtransfusie, Amsterdam, The Netherlands) is dissolved in TNP buffer to give a stock solution of 835 nKat·ml⁻¹. Immediately before use this solution is diluted with TNP buffer to give a concentration of 3.34 nKat·ml⁻¹.
- * All ingredients used are of analytical grade
- For aqueous solutions ultrapure water (Milli-Q quality) is used.

### Preparation of test and reference compound solutions

The test and reference compounds are dissolved in Milli-Q water to give stock concentrations of 10⁻² mol·l⁻¹. Each concentration is stepwise diluted with the vehicle to give concentrations of 10⁻³, 10⁻⁴ and 10⁻⁵ mol·l⁻¹. The dilutions, including the stock solution, are used in the assay (final concentrations in the reaction mixture: 3·10⁻³; 10⁻³; 3·10⁻⁴; 10⁻⁴; 3·10⁻⁵; 10⁻⁵; 3·10⁻⁶ and 10⁻⁶ mol·l⁻¹, respectively).

### Procedure

At room temperature 0.075 ml and 0.025 ml test compound or reference compound solutions or vehicle are alternately pipetted into the wells of a microtiter plate and these solutions are diluted with 0.115 ml and 0.0165 ml TNP buffer, respectively. An aliquot of 0.030 ml S-2238 solution is added to each well and the plate is pre-heated and pre-incubated with shaking in an incubator (Amersham) for 10 min. at 37 °C. Following pre-incubation the hydrolysis of S-2238 is started by addition of 0.030 ml thrombin solution to each well. The plate is incubated (with shaking for 30 s) at 37 °C. Starting after 1 min of incubation, the absorbance of each sample at 405 nm is measured every 2 min. for a period of 90 min. using a kinetic microtiter plate reader (Twinreader plus, Flow Laboratories).
All data are collected in an IBM personal computer using LOTUS-MEASURE. For each compound concentration (expressed in mol·l⁻¹ reaction mixture) and for the blank the absorbance is plotted versus the reaction time in min.

Evaluation of responses: For each final concentration the maximum absorbance was calculated from the assay plot. The IC₅₀-value (final concentration, expressed in µmol·l⁻¹, causing 50% inhibition of the maximum absorbance of the blank) was calculated using the logit transformation analysis according to Hafner et al. (Arzneim.-Forsch./Drug Res. 1977; 27(II): 1871-3).

## Claims

1. A compound having the formula I 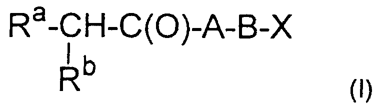 wherein R^{a} is -NR¹R² and R^{b} is R³; R¹ is -CH₂COOH or -CH₂CH₂COOH;
R₂ is H; R₃ is -CH₂-(3-8C)cycloalkyl; A is proline, 3,4-dehydroproline or azetidine carboxylic acid; B is lysine and X is -COOH or a pharmaceutically acceptable salt thereof.

2. The compound of claim 1, being HOOC-CH₂-D-cyclohexylalanylprolyl-lysyl-COOH.

3. A pharmaceutical composition comprising the compound of claim 1 or 2 and pharmaceutically suitable auxiliaries.

4. The compound of claim 1 or 2 for use in therapy.

5. Use of the compound of claim 1 or 2 for the manufacture of a medicament for treating or preventing thrombin-related diseases.

## Patentansprüche

1. Eine Verbindung mit der Formel I, 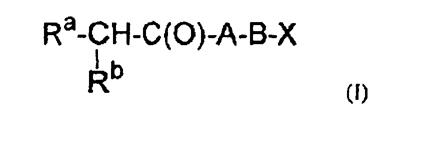 wobei R^{a} gleich -NR¹R² und R^{b} R³ ist; R¹ -CH₂COOH oder -CH₂CH₂COOH ist; R² H ist; R³ -CH₂-(3-8C)cycloalkyl ist; A Prolin, 3,4-Dehydroprolin oder Azetidin-Carbonsäure ist; B Lysin ist und X -COOH oder ein pharmazeutisch verträgliches Salz davon ist.

2. Die Verbindung nach Anspruch 1, die HOOC-CH₂-D-Cyclohexylalanyl-Prolyl-Lysyl-COOH ist.

3. Eine pharmazeutische Zusammensetzung mit der Verbindung nach Anspruch 1 oder 2 und pharmazeutisch geeignete Hilfsstoffe.

4. Die Verbindung nach Anspruch 1 oder 2 zur Verwendung in einer Therapie.

5. Verwendung der Verbindung nach Anspruch 1 oder 2 zur Herstellung eines Medikamentes zur Behandlung oder Vermeidung von Thrombin-bezogenen Krankheiten.

## Revendications

1. Composé de formule I 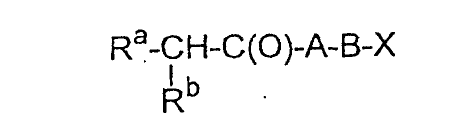 dans laquelle
- R^{a} est -NR¹R² et R^{b} est R³;
- R¹ est -CH₂ COOH ou -CH₂CH₂COOH;
- R² est H;
- R³ est -CH₂-(C₃-C₈)cycloalkyle ;
- A est la proline, la 3,4-déhydroproline ou l'acide azétidine-carboxylique;
- B est la lysine et X est -COOH
ou un de ses sels pharmaceutiquement acceptable.

2. Le composé selon la revendication 1, consistant en HOOC-CH₂-D-cyclohexylalanyl-prolyl-lysyl-COOH.

3. Une composition pharmaceutique comprenant le composé selon la revendication 1 ou 2 et des additifs pharmaceutiquement acceptables.

4. Le composé selon la revendication 1 ou 2 pour une utilisation thérapeutique.

5. Utilisation du composé selon la revendication 1 ou 2 pour la fabrication d'un médicament pour traiter ou prévenir les maladies liées à la thrombine.
